Europäisches Patentamt

European Patent Office

Office européen des brevets

(11) Publication number: **0 491 076 A1**

# EUROPEAN PATENT APPLICATION

(21) Application number: **90124767.6**

(22) Date of filing: **19.12.90**

(51) Int. Cl.5: **A61K  47/10**, **A61K 47/22**

(43) Date of publication of application:
**24.06.92 Bulletin  92/26**

(84) Designated Contracting States:
**AT BE CH DE DK ES FR GB GR IT LI NL SE**

(71) Applicant: **THERATECH, INC.**
**Research Park 410 Chipeta Way Suite 219**
**Salt Lake City Utah 84108(US)**

(72) Inventor: **Patel, Dinesh C.**
**5839 Meadow Crest Drive**
**Murray, Utah 84107(US)**
Inventor: **Ebert,Charles D.**
**558 N. Perry's Hollow**
**Salt Lake City, Utah 84103(US)**
Inventor: **Cheng, Daniel C.**
**3288 Walnut Way**
**Salt Lake City, Utah 84121(US)**
Inventor: **Heiber, Werner E.**
**1464 Wilton Way**
**Salt Lake City, Utah 84108(US)**

(74) Representative: **Patentanwälte Grünecker,**
**Kinkeldey, Stockmair & Partner**
**Maximilianstrasse 58**
**W-8000 München 22(DE)**

(54) **Penetration enhancement with multi-component system of N-aliphatic pyrrolidones with lower alcohols.**

(57) Penetration-enhancing pharmaceutical compositions for topical transepidermal and percutaneous application are disclosed which are non-irritating to the skin. These compositions are made up of a safe and effective amount of an active pharmaceutical permeant, including hydrophilic salt forms, contained in a novel penetration-enhancing vehicle comprising, (1) 0.01 to 20% w. of an N-aliphatic pyrrolidone, wherein the aliphatic portion is a $C_8$ to $C_{22}$ alkyl or alkenyl chain which can be a straight or branched; (ii) 5-75% w. of a lower alkanol selected from the group consisting of ethanol, propanol and isopropanol and mixtures thereof; (iii) 5.0 to 94.99% w. an inert carrier such as water and (iv) 0 to 20% w. of a cell-envelope disordering compound, such as methyl laurate, glycerol monolaurate, oleic acid, oleyl alcohol, glycerol monooleate, glycerol dioleate, glycerol trioleate and mixtures thereof. Capryl, lauryl, myristoleyl and oleyl pyrrolidones are exemplary of the N-alkyl or alkenyl pyrrolidones which may be utilized. The compositions may be administered to humans or warm-blooded animals by being applied directly to the skin or, preferably, administered in the form of a sustained release device such as a patch.

This invention relates to compositions which enhance the penetration of pharmaceutically-active agents through the integument. More particularly, this invention relates to multi-component combinations of certain penetration enhancers which facilitate percutaneous and transepidermal delivery of a broad range of pharmaceutically-active agents.

The resistance of the skin to being penetrated by pharmaceutically-active agents is well documented. As compared to mucosal tissues, the stratum corneum is compact and highly keratinized and quite impermeable. Such impermeability of the skin is highly essential to the well being of a living organism in that it serves as a barrier to the ingress of pathogens and toxic materials, and the egress of physiologic fluids.

The impermeability of pharmaceutical agents through the skin is due to the nature of the very thin stratum corneum layer which is only 10-15 cells, i.e. about 10 microns thick. This layer is formed naturally by cells migrating toward the skin surface from the basal layer. Cells slowly move from the basal layer to the surface where they are sloughed off. As they progress toward the surface they become progressively more dehydrated and keratinized.

Because of the advantages of dermal application of pharmaceutically-active agents, various penetration enhancers have been sought. A penetration enhancer is one or more compounds which alter the skin as a barrier to increase the flux of a desired pharmaceutical permeant across the skin.

Penetration enhancers have been primarily categorized according to their ability to enhance permeant flux via three pathways. The first is the continuous polar or aqueous pathway composed of proteins. It is thought that solvent swelling or protein conformational changes provide the key to altering the penetration of the polar pathway. Surfactants alter the transport of polar permeant molecules to a much greater extent than the transport of nonpolar permeants. Solvents such as DMSO, 2-pyrrolidone and dimethylformamide can swell the stratum corneum to also enhance the polar pathway.

The second pathway is a continuous non-polar pathway consisting of lipids. The key to altering this pathway appears to be fluidizing the lipids which, in the stratum corneum, appear to be crystalline. Solvents such as DMSO, 2-pyrrolidone, and dimethylformamide, previously mentioned also appear able to solubilize or fluidize lipids. Other solvents include diols such as glycerol and propylene glycol.

The third pathway is a heterogeneous polar-nonpolar multilaminate of lipids and proteins.

Prior art binary systems consist of a particular category of a polar solvent combined with a variety of compounds generally referred to as "cell-envelope disordering compounds".

U.S. Patent 4,537,776, Cooper, issued August 27, 1985, contains an excellent summary of prior art and background information detailing the use of certain binary systems for permeant enhancement. Because of the completeness of that disclosure, the information and terminology utilized therein are incorporated herein by reference. That patent teaches using a binary system wherein N-(2-hydroxyethyl) pyrrolidone is used as the solvent and the cell-envelope disordering compounds are selected from the group consisting of methyl laurate, oleic acid, oleyl alcohol, moloolein, myristyl alcohol and mixtures thereof.

Similarly, European Patent Application 43,738, published January 13, 1982, teaches using selected diols as solvents along with a broad category of cell-envelope disordering compounds for delivery of lipophilic pharmacologically-active compounds. This reference also teaches that cosmetically acceptable solvents may also be combined with the permeant and the diol and cell-envelope disordering compounds to dissolve the active components provided the solvent evaporates rapidly and completely to leave only the active components of the composition at the site of application. The acceptable solvents are stated to be ethanol or isopropanol. Because of the detail in disclosing the cell-envelope disordering compounds and the diols, the disclosure of European Patent Application 43,738 is also incorporated herein by reference.

Most of the cell-envelope disordering compounds mentioned in these publications are unsaturated lipid components having polar head groups.

A binary system for enhancing metoclopramide penetration is disclosed in UK Patent Application GB 2,153,223 A, published August 21, 1985 and consists of a monovalent alcohol ester of a C8-32 aliphatic monocarboxylic acid (unsaturated and/or branched if C18-32) or a C6-24 aliphatic monoalcohol (unsaturated and/or branched if C14-24) and an N-cyclic compound such as 2-pyrrolidone, N-methylpyrrolidone and the like. It is postulated that the N-cyclic compound serves a solvent function which carries the active agent whereas the esters or alcohols serve as adjuvants to open up the stratum corneum, i.e. as cell-envelope disordering compounds.

Sato, U.S. Patent 4,593,048, issued June 3, 1986, shows skin permeation of drugs using a system consisting of a major amount of a lower alcohol having 1 to 4 carbon atoms and a minor amount of an adjuvant consisting of (1) a saturated straight chained, branched chained or cyclic aliphatic hydrocarbon containing 5 to 20 carbon atoms which may be optionally halogen substituted, (2) a carboxylic acid ester containing 13 to 24 carbon atoms wherein the acid moiety contributes 12 to 18 carbon atoms and the

2

alcohol moiety is from 1 to 6 and preferably from 3 to 6 carbon atoms and (3) an ether containing 8 to 16 carbon atoms. The adjuvant is incorporated in amounts ranging from 1 to 50 percent by weight, and preferably 5 to 30 percent by weight, based on the lower alcohol.

Patel et al., allowed copending application Serial No. 07/218,702, filed July 13, 1988 as a continuation-in-part of Serial No. 06/930,764, filed November 14, 1986, teaches penetration-enhancing compositions for topical application comprising an active pharmaceutical contained in a penetration-enhancing vehicle made up of 1-95% by weight of one or more cell-envelope disordering compounds selected from the group consisting of oleic acid, oleyl alcohol, glycerol esters of oleic acid and mixtures thereof; 5-75% by weight of a $C_2$ or $C_3$ alkanol and 0-45% by weight of an inert diluent such as water.

Patel et al., allowed copending application Serial No. 07/240,688, filed September 9, 1988, discloses compositions for reducing skin irritation caused by drug/enhancer compositions having skin irritation properties comprising a percutaneously absorbable drug, a mild to moderately irritating binary enhancer composition consisting of (a) a solvent selected from the group consisting of a $C_2$ or $C_3$ alcohol, a $C_3$ or $C_4$ diol, DMSO, DMF, DMA, 1-n-dodecyl-cyclazacycloheptan-2-one, N-methyl-pyrrolidone and N-(2-hydroxyethyl)pyrrolidone and mixtures thereof and (b) an effective enhancing amount of a cell envelope disordering compound, and an amount of glycerin sufficient to provide an anti-irritating effect.

Sanders, U.S. Patent 4,764,379, issued August 16, 1988, teaches a binary enhancer combination of glycerol monolaurate and ethanol. The crux of the Sanders patent is that the actual flux attained with these combined enhancers is significantly greater than the sum of the drug fluxes obtained with the individual enhancer, i.e. the theoretical flux.

Kigasawa et al., U.S. Patent 4,695,465, issued September 22, 1987 mentions dodecyl pyrrolidone, along with a host of other ingredients, as an absorption promoting auxiliary on column 7, lines 17 and 18 but provides no support for this statement.

Sasaki et al., Int. J. of Pharm. 44 (1988) pp. 15-24, suggests the use of N-alkyl pyrrolidones as penetration enhancers although no supporting data are given.

Other patents or publications relating to transdermal administration of active permeants are Cooper, European Patent Application 95,813,A2, published July 12, 1983, entitled Penetrating Topical Pharmaceutical Compositions Containing 9-(2-Hydroxyethoxymethyl)Guanine; Durrant et al, European Patent Application 117,080, published August 29, 1984, entitled Skin Treatment Composition.

## SUMMARY OF THE INVENTION

The present invention relates to improved compositions and methods for improving the penetration of a broad category of pharmaceutically-active agents which are lipophilic or hydrophilic including salts and which produce little or no skin irritation to human or animal tissue systems. The invention provides penetrating topical compositions based on the use of a pharmaceutically-active agent dissolved in, or admixed with, a penetration-enhancing multi-component mixture of (a) about 0.01 to 20% by weight of one or more N-aliphatic pyrrolidones and (b) 5-75%, by weight of a $C_2$ or $C_3$ lower alcohol and (c) 5.0 to 94.99% by weight of an inert carrier such as water. The aliphatic portion of the pyrrolidone is a $C_8$ to $C_{22}$ alkyl or alkenyl chain which may be either straight or branched. The formulation can optionally contain between 0 to 20% by weight and preferably between about 0.01 to 20% by weight, of one or more cell-envelope disordering compounds such as methyl laurate, glycerol monolaurate, oleic acid, oleyl alcohol, glycerol mono-, di- or trioleate and mixtures thereof. However other cell envelope disordering compounds such as disclosed in U.S. Patent Nos. 4,537,776 and European Patent Application 43,738, published January 13, 1982, cited above and incorporated herein by reference may also be utilized. In addition to or in the place of water may be used other inert ingredients which are soluble within the enhancer composition. Such ingredients can vary from hydrophilic to hydrophobic depending upon the desired combination. Representative inert ingredients other than water include glycerin, polypropylene glycol, polyethylene glycol, polyvinyl alcohols, polyvinylpyrrolidone, mineral oil, silicone oil, ethylene-vinyl acetate polymers or other low molecular weight polymers soluble in water, lower alcohols or suitable oils.

By employing this multi-component mixture with or without optional cell-envelope disordering compounds, it has been found that significant penetration of salts and other hydrophilic permeants as well as lipophilic permeants is obtained and that skin irritation often associated with cell-envelope disordering compounds and/or solvents is essentially nonexistent.

The invention is therefore not limited to any specific category or categories of permeants but is inclusive of all therapeutically active compounds and their use which are responsive by being incorporated into the multi-component mixture as more fully set forth herein.

Also, the invention is drawn to treatment methods by means of which an effective amount of a permeant, combined with the multi-component mixture, is topically applied to a human or animal subject.

DETAILED DESCRIPTION OF THE INVENTION

The following definitions, when used and as they apply to the present invention, are consistent with those contained in U.S. Patent 4,537,776.

By "topical administration" or "topical application" is meant directly laying or spreading upon epidermal tissue, especially outer skin or membrane, including the skin or membrane of the oral, nasal, occular, anal or vaginal cavities.

By "safe and effective", is meant a sufficient amount of the permeant composition to provide the desired systemic effect and performance, or local activity, or both at a reasonable benefit/risk ratio attendant any medical treatment. Within the scope of sound medical judgment, the amount of permeant used will vary with the particular condition being treated, the severity of the condition, the duration of the treatment, the specific permeant compound employed, its concentration, the condition of the patient, concurrent therapies being administered and other factors within the knowledge and expertise of the patient or the attending physician or other practitioner.

By "toxicologically- or pharmaceutically-acceptable" is meant the pharmaceutical actives (or permeants), as well as the other compatible drugs, medications or inert ingredients which the term describes, are suitable for use in contact with the tissues of humans and animals without undue toxicity, irritation, allergic response, and the like commensurate with a reasonable benefit/risk ratio.

By the terms "comprising" is meant that various other compatible drugs and medicaments, as well as inert ingredients, occlusive agents, and cosmetic vehicles, can be conjointly employed in the compositions and methods of this invention, as long as the critical multi-component penetration enhancement vehicle and pharmaceutically active permeant are used.

By "afflicted situs" is meant a localized area of pathology, discomfort, infection, inflammation or lesion, and the immediately surrounding area.

By "application situs" is meant a site suitable for topical application with or without the means of a mechanical sustained release device, patch or dressing, e.g. behind the ear, on the arm, back, chest, stomach, leg, top of foot, etc.

By "device" or "sustained release device" is meant a physical object adhering and/or otherwise contacting the skin and/or mucosa which coadministers a permeant and enhancer to the application situs.

By "penetration-enhancing" is meant that the multi-component penetration enhancing carriers or vehicles of this invention with or without optional inert ingredients provide marked transepidermal or percutaneous delivery of an incorporated active permeant, when compared to other compositions at equal chemical potential. Equal chemical potential is important since varying solubilities of drugs in different carrier vehicles will affect their transport across skin. As stated in U.S. Patent 4,537,776, if a drug is soluble in vehicle A to the extent of 24%, and in vehicle B to the extent of 4%, were the compositions to be compared at equal percentage concentration, rather than equal chemical potential, the lower solubility carrier would show a misleading six-fold difference in transport over the more soluble vehicle. Therefore, the simplest way of assuring equal chemical potential for evaluating penetration enhancement is to use saturated solutions or solutions of equal percentage of saturation of active permeants in the various enhancer combinations, e.g. 50% saturated. In the examples used herein, unless stated otherwise, the enhancer combinations are saturated with the active permeant components.

As used herein, all percentages and ratios are by weight of the total composition unless otherwise specified.

The terms "permeant", "active", "pharmaceutical active", "pharmacological active", "pharmaceutical agent", "pharmacological agent", "pharmaceutically-, or pharmacologically-active agent", "chemical agent", "therapeutic agent", and "drug", are used interchangeably herein.

The compositions of this invention require, at a minimum, a permeant capable of producing systemic effects, or producing or possessing local activity, in a multi-component vehicle comprising an N-$C_8$ to $C_{22}$ aliphatic (alkyl or alkenyl) pyrrolidone, a $C_2$ or $C_3$ alcohol, an inert carrier and, optionally, a cell-envelope disordering compound preferably selected from the group consisting of methyl laurate, glycerol monolaurate, oleic acid, oleyl alcohol, glycerol monooleate, glycerol dioleate, glycerol trioleate and mixtures thereof with or without other optional ingredients within the stated ranges.

The composition may also contain other optional components which enhance their cosmetic appeal or acceptability, i.e., thickeners, pigments, fragrances, perfumes, and the like. The multi-component penetration combinations are essentially free of skin irritation characteristics. However, a permeant combined with

4

the penetration enhancers may cause some irritation. Therefore, if desired, components such as glycerin, which tend to reduce skin irritation may be incorporated into the compositions.

MULTI-COMPONENT PENETRATION ENHANCEMENT VEHICLES

The multi-component penetration enhancement combinations of the present invention significantly enhance the penetration of a host of pharmaceutically-active permeants including salts. These permeants may be lipophilic or hydrophilic or partially lipophilic or hydrophilic.

The multi-component combinations comprise one or more of the stated N-alkyl or alkenyl pyrrolidones, a lower alkanol selected from the group consisting of ethanol, propanol and isopropanol and an inert carrier with or without the optionally stated cell-envelope disordering compounds and other optional ingredients.

The N-alkyl pyrrolidones are surfactants some of which are known to function in shampoo formulations. See for example, Helioff et al, Shampoo Innovation Via A New Surfactant, D & Cl., April 1988, pp 38-42 which shows N-octyl pyrrolidone (capryl pyrrolidone) and N-dodecyl pyrrolidone (lauryl pyrrolidone) as shampoo surfactants having low skin irritation properties. These are marketed by GAF Chemicals Corporation under the tradenames Sulfadone LP-100 and Sulfadone LP-300 respectively. Additionally, myristoleyl ($C_{14}$) and oleyl ($C_{18}$) pyrrolidones have been synthesized. Alkyl or alkenyl groups of from $C_8$ through $C_{22}$ are functional in the present invention with $C_{12}$ to $C_{18}$ alkyl groups being preferable. These N-alkyl or N-alkenyl pyrrolidones, hereinafter also referred to by the acronym NAP, may be present in amounts ranging from between about 0.01 to 20.0 %w. of the multi-component enhancer formulation with ranges of between about 0.1 and 5.0 %w. being preferable and ranges of between about 0.1 to 2.0 %w. being most preferable. It is not necessary to have larger amounts since, as will be shown below, 1% w. NAP formulations in 99% water show penetration enhancement effects comparable to 100% w. NAP. Thermodymically 1% NAP in 99% water is equivalent to 100% NAP. In other words, 1 %w. aqueous solutions of NAP are saturated.

The bulk of the enhancer formulation is preferably a mixture of lower alkanol, preferably ethanol, and inert carrier, preferably water. The concentration of the lower alcohols generally ranges between about 5 and 75% by weight of the penetration enhancer combination. At concentrations above about 50% by weight lower alcohols can cause skin irritation and one may also become sensitized to the alcohols. Therefore, it is desirable that a portion of the enhancer be made up of water or other suitable inert carrier. However, alcohol concentrations up to about 75% w. are functional and may be used particularly since the NAP ingredients possess low skin irritation properties. Alcohol concentrations of between about 5 and 70% w. are preferable with concentrations between about 10 and 60% w. being most preferred. Inert carrier concentrations generally vary between about 5.0 and 94.99 %w. with concentrations between about 5.0 and 70.0% w. being preferable and concentrations bewteen about 20 and 70% being most preferred.

When present, cell envelope disordering compounds are used in amounts ranging between about 0.01 and 20 %w and preferably between about 0.1 and 5.0 %w. and ranges of between about 0.1 to 2.0 %w. being most preferable. In the prior art, these ingredients are often used in much higher concentrations. However, it is not necessary to exceed the above ranges to achieve maximum penetration enhancement effects.

The enhancer combinations are referred to as "multi-component" since NAP, lower alcohols and inert carrier(s) are required components. It may be preferable, for reasons given above, to combine one or more inert components when it is desired to maximize the effectiveness of the NAP and lower alcohol with any given active permeant and retain the content of the lower alcohol within the desired range of 75%, and preferably 50%, and below. In most instances, the inclusion of water is sufficient. Other inert carriers can be any compatible ingredient which is soluble in the NAP-lower alcohol-inert carrier enhancer combination with or without the presence of the cell-envelope disordering compound. Such ingredient may be hydrophilic or hydrophobic depending upon the enhancer combination and the active permeant to be used. The invention is not limited to any particular inert ingredient as such will be readily ascertainable by one skilled in the art. Typical of such inert ingredients, other than water, are glycerin, polypropylene glycol, polyethylene glycol, polyvinyl alcohols, polyvinylpyrrolidone, mineral oil, silicone oil, ethylene-vinyl acetate polymers or other low molecular weight polymers soluble in water, lower alcohols or suitable oils.

The compositions of the invention typically contain from about 50 to 99.9999%, and preferably about 70 to 99.99%, by weight of the overall enhancer composition mixture comprising the N-alkyl or alkenyl pyrrolidone, a lower $C_2$ or $C_3$ alcohol and inert carrier, an optional cell-envelope disordering compound and inert ingredients within the ranges defined herein. The exact amount of active permeant, and the range of each ingredient used in the enhancer combination may be readily determined by one having ordinary skill in the art. All that is required is that an effective amount of the active permeant be incorporated into the penetration enhancing composition as described, with or without the present of other optional ingredients.

PHARMACEUTICALLY-ACTIVE PERMEANTS

The multi-component penetration enhancers of the present invention may be formulated to incorporate a broad range of pharmaceutically-active permeants. One of the distinct advantages of these enhancer combinations is that they function to enhance penetration of both lipophilic and hydrophilic permeants including salts and are virtually free from skin irritation effects. The compositions of this invention may be utilized in delivering active permeants to the "target" areas as mentioned in U.S. Patent 4,537,776, i.e. (1) at the surface of the skin; (2) in the stratum corneum itself; (3) in the viable epidermis and upper dermis, just below the stratum corneum; (4) in the various glands and structures in and beneath the dermis (e.g., subcutaneous adipose, dermal vasculature); and/or (5) the general system (i.e. systemic effects).

In view of this, the invention is not limited to any specific type or class of active permeants. Based on the parameters contained herein it is within the ability of one having ordinary skill in the art to determine which permeants can be utilized. Some routine experimentation or testing may be required to determine optimum conditions such as exact concentrations of permeants, ratios of NAP compounds to alcohols, inert carriers and the like. Also, some permeants may work best with one particular class of alkyl or alkenyl pyrrolidone and/or alcohols. The screening of all possible combinations and ratios of permeants, N-aliphatic pyrrolidones, alcohols, inert carriers and cell-envelope disordering compounds and has not been attempted.

However, based on the formulation of a representative sampling of diverse active permeants, it is apparent that the multi-component combination of N-alkyl or N-alkenyl pyrrolidones a $C_2$ or $C_3$ alcohol and inert carrier, with or without cell-envelope disordering compounds or optional ingredients, will function to enhance the penetration of a broad spectrum of pharmaceutically-active permeants. Such agents include, without limitation, those mentioned in U.S. Patent 4,537,776 such as antimicrobials, antibacterials, antibiotics, antimyobacterials, antimalarials, antiamebics, anthelmintics, antifungals, antivirals, neoplastic agents, agents affecting the immune response, blood calcium regulators, peptide and protein hormones, male sex hormones, female sex hormones, agents useful in glucose regulation, anticoagulants, antithrombotics and hemostatics, antihyperlipidemic agents, cardiac drugs, thyromimetic and antithyroid drugs, adrenergics, antihypertensive agents, cholinergics, anticholinergics, antispasmodics, antiulcer agents, skeletal and smooth muscle relaxants, histamine $H_2$-receptor agonists and antagonists, dopamine receptor agonists and antagonists, prostaglandins, general inhibitors of the allergic response, antihistamines, local anesthetics, analgesics, narcotic antagonists, antitussives, sedative-hypnotic agents, anticonvulsants, antipsychotics, anti-anxiety agents, antidepressant agents, anorexigenics, non-steroidal anti-inflammatory agents, steroidal anti-inflammatory agents, bone-active agents, antiarthritics, polypeptides, vitamins, diagnostic agents and sunscreens. These agents can be used for systemic effect, local activity, or both, as appropriate. Examples of pharmaceutically-active permeants are well-known in the art and can be found listed in sources identified in U.S. Patent 4,537,776 as well as others. For example, active agents, in approved commercially available formulations, their recommended dosages, adverse reactions, side effects and the like are listed in the annual publication of the Physicians' Desk Reference, published by Medical Economics Company, a division of Litton Industries, Inc.

The pharmaceutically-active permeants may be used in the compositions and methods of the present invention at any safe and effective level, or in any safe and effective amount. Dosages will obviously be a function of various variables, such as how active the agent is, how soluble it is in the penetration enhancing composition, how often it is to be applied, whether the use is to be topical (applied to the "afflicted situs") or systemic (applied to the "application situs"), whether two or more active permeants are to be combined, the particular patient being treated, and the like. In any event, the dosage will be the smallest that will achieve the desired result and the period of administration will be as short as possible to attain this result.

In general, dosages and means of application as taught in U.S. Patent 4,537,776 are appropriate to the present invention. Levels of active permeants may vary from about 0.0001% to about 50% by weight of the total composition with levels of from about 0.01 to 30% being preferred. Levels from about 0.05 to 15% being especially preferred and levels of from about 0.1 to 10% being most especially preferred for some active permeants. However, for some active permeants, it may be required to use more or less than stated above to attain the desired results. Hence, the invention is not directed to any particular amount of active ingredient as long as it is safe and effective.

A compendium of active permeants is contained in U.S. Patent 4,537,776 and published European Patent Application 43,738 and incorporated herein by reference. However, for purposes of illustration, a concise listing of active agents follows.

Typical antihypertensive agents which may be utilized include, without limitation, minoxidil, nadolol, pargyline, pindolol, propranolol, reserpine, timolol, trimethaphan, metoprolol, hydrochlorothiazide, hydralazine, furosemide, clonidine, and chlorthalidone.

Diuretics include, without limitation, benzthiazide, buthiazide, cyclopenthiazide, cyclothiazide, metolazone, triamterelene, chlorazanil, clazolimme, and hydroflumethiazide.

Exemplary of anorexigenics are, without limitation, amphetamine, methamphetamine, chlorphentermine, chlortermine, phentermine, phendimetrazine, mazindol, oxazoline, and phenoxyalbyleneamine.

Fungistatic and fungicidal agents encompass, without limitation, thiabendazole, chloroxine, fungimycin, griseofulvin, chlordantoin, salicylic acid, nystatin, clotrimazole, fezatione, sodium pyrithione, amphotericin B, 5-fluorocytosine, haloprogin, vifampin, and pimaricin.

A broad range of analgesics may be utilized including, without limitation, morphine, codeine, heroine, methadone, thebaine, orpiarine, buprenorphine, morphinans, benzomorphans, acetaminophen, butorphanol, diflunisal, fenoprofen, fentanyl, fentanyl citrate, hydrocodone, aspirin, sodium salicylate, ibuprofen, oxymorphone, pentaxicine, naproxen, nalbuphine, mefenamic acid, meperidine and dihydroergotamine.

A typical narcotic antagonist is haloxone. Exemplary antitussive agents include, without limitation, diphenhydramine, guaifenesin, hydromorphone, ephedrine, phenylpropanolamine, theophylline, codeine, noscapine, levopropoxyphene, carbetapentane, chlorpehndianol and benzonatate.

Among the sedatives which may be utilized are, without limitation, chloral hydrate, butabarbital, alprazolam, amobarbital, chlordiazepoxide, diazepam, mephobarbital, secobarbital, diphenhydramine, ethinamate, flurazepam, halazepam, haloperidol, prochlorperazine, oxazepam, and talbutal.

Examples of cardiac drugs are, without limitation, quinidine, propranolol, nifedipine, procaine, dobutamine, digitoxin, phenytoin, sodium nitroprusside, nitroglycerin, verapamil HC1, digoxin, nicardipine HC1, captopril, and isosorbide dinitrate.

Antimicrobial agents are inclusive of, without limitation, erythromycin, sulfonamide, lincomycin, clindamycin, tetracycline, chlortetracycline, demeclocycline, doxycycline, and methacycline.

Examples of useful antibacterial agents are, without limitation, phenols, hydroxy benzoic acid, hydroxy quinoline, nitrofuran, nitroimidazoles, oxolinic acid, actinomycetin, bacitracin, tyrothricin, kanamycin, neomycin and chloramphenicol.

A typical antiviral agent is Ara-A. Steroidal anti-inflammatory agents are illustrated by, without limitation, triamcinolone acetonide, beclomethasone dipropionate, hydrocortisone acetate, fluocinolone acetonide, betamethasone valerate, prednisolone, prednisone, methyl prednisolone and paramethasone.

Inclusive of non-steroidal anti-inflammatory agents are, without limitation, acetyl salicylic acid, fenoprofen calcium, ibuprofen, ketoprofen, indomethacin, meclofenamate sodium, mefenamic acid, naproxen sodium, phenylbutazone, and oxyphenbutazone, diclofenac sodium and piroxicam.

Anti-emetics are illustrated by, without limitation, thiethylperazine, metoclopramide, cyclizine, meclizine, prochlorperazine, doxylamine succinate, promethazine, triflupromazine, and hydroxyzine.

A typical dopamine receptor agonist is bromocriptine mesylate. Exemplary amino acid, peptide and protein hormones include, without limitation, thyroxine, growth hormone (GH), interstitial cell stimulating hormone (ICSH), follicle-stimulating hormone (FSH), thyrotropic hormone (TSH), adrenocorticotropic hormone (ACTH), gonadotropin releasing hormone (GnRH) such as leuprolide acetate, vasopressin and their active degradation products. Some products may have sufficiently high molecular weights that absorption through the stratum corneum or mucous membranes may be difficult. Therefore, the invention is applicable only to those hormones which have molecular weights and stereo configurations which will allow passage through the skin.

Female sex hormones which can be used include, without limitations, estradiol, diethylstilbestrol, conjugated estrogens, estrone, norethindrone, medroxyprogesterone, progesterone, and norgestrel.

Typical male sex hormones which may be utilized may be represented by, without limitation, testosterone, methyltestosterone, and fluoxymesterone.

The above listed active permeants may, along with others not specifically disclosed, be used separately or in combination according to the treatment regimen desired.

Preferred categories of active permeants include anti-hypertensive agents, cardiac drugs, analgesics, sedative-hypnotic agents, anti-anxiety agents, steroidal anti-inflammatory agents, non-steroidal anti-inflammatory agents, male sex hormones, and female sex hormones. Those active permeants specifically listed above under each category are particularly preferred.

The components of this invention are inclusive of the active permeants combined with the multi-component penetration enhancing mixture of N-alkyl or alkenyl pyrrolidones, $C_2$ and $C_3$ alcohols and inert carrier(s) with or without the addition of cell-envelope disordering compounds and optional ingredients. It is contemplated that compositions containing only these ingredients will be sufficient in most instances to obtain the desired results. However, in preparing formulations for actual use, it may be desirable, in addition to inert carriers, to add other components such as excipients, dyes, perfumes fragrances, pacifiers, thickening agents, preservatives, anti-oxidants, gelling agents, surfactants and stabilizers. Such materials, when added, should not unduly interfere with the penetration enhancement of these compositions. Such formula modifications to improve cosmetic acceptability are well within the skill of workers in the art and do not form part of the present invention.

METHOD OF USE

In any form of medical practice, there are many variables which affect the particular treatment regimen. In that regard, the final diagnosis and treatment is left to the expertise of the practitioner and patient. As previously stated, in clinical practice, it is the goal that the dosage of any active permeant be as small as possible to achieve the result desired. To attain these conditions, it is imperative that the amount of active ingredient utilized is a safe and effective amount whether applied to an afflicted situs or an application situs. When local treatment is desired, the compositions are applied to the afflicted situs. When systemic treatment is desired, the compositions are applied to an application situs, preferably from a sustained release device such as a patch, bandage, web, film or the like. Such devices are commonly in the form of a laminated composite that includes a reservoir layer containing the permeant or drug, a pressure sensitive adhesive layer for attaching the composite to the skin, and a backing layer that forms the upper layer of the device. Depending upon the particular drug and drug formulation involved, the reservoir layer may be a matrix in which the drug formulation is dispersed or a layer in the form of a walled container which holds the drug formulation. Container-type reservoirs are often formed as a pocket between the backing layer and a drug-permeable basal membrane through which the drug passes to the skin. Typical sustained release devices which can be utilized are disclosed in Kwiatek et al., U.S. Patent 4,573,996; Nuwayser, U.S. Patent 4,687,481; Sanders et al, U.S. Patent 4,764,379 and Chang et al., U.S. Patent 4,849,224. When both local and systemic treatments are indicated, the compositions can be applied at both the afflicted situs and application situs, or both. The selection of active permeant or combination of permeants, particular penetration enhancement combination and the like are necessarily left to the skill of the practitioner provided the parameters outlined herein are followed.

The dosage, rate of application, place of application, and other treatment parameters are generally outlined in U.S. Patent 4,537,776 and are incorporated herein by reference rather than being repeated. What is a safe and effective amount of any ingredient will obviously depend upon the active ingredient being used, the site of application, the effectiveness of the penetration enhancer and other parameters outlined herein.

A practitioner being skilled in the art will be able to determine the application parameters of each specific formulation based on the needs of each patient.

EXAMPLES

In order to demonstrate the effectiveness of the present invention, the following examples demonstrate the penetration enhancement which is obtained by utilizing individual enhancement components separately or in water, combinations of components from prior art teachings as well as the multi-component N-alkyl or alkenyl pyrrolidone-lower alkanol-water compositions of the present invention. In making these tests, human skin consisting of heat-separated abdominal epidermis, taken at autopsy, was placed in a standard Franz diffusion apparatus in a horizontal position between a lower closed diffusion cell and an upper capped diffusion cell. A normal saline solution was added to the lower diffusion cell in contact with the subcutaneous side of the skin. The test composition, consisting of a saturated (unless otherwise indicated) solution of an active drug being tested formulated in the multi-component enhancer, was added to the diffusion cell in contact with the upper or epidermal side of the skin.

The cell assembly was kept at a constant temperature of 32°C. At predetermined intervals, the diffusate from the cell on the subcutaneous side of the skin was withdrawn and the amount of drug in the diffusate was measured using standard analytical techniques. Each test was run using a separate skin sample. Unless stated differently, the amount of active drug used was that required to form a saturated solution. The results are reported in terms of flux [ug/cm2/day (hour)].

EXAMPLE I

This example shows the pindolol flux obtained using a saturated solution of pindolol in the individual enhancer components separately and, in the case of water and ethanol, in combination.

| Test No. | Enhancer Ingredient | Flux [ug/cm2/hour] |
|---|---|---|
| I-A | 100% Ethanol | 3.5 |
| I-B | 100% Water | 1.6 |
| I-C | 50%/50% Ethanol/Water | 3.9 |
| I-D | 100% Methyl Laurate (ML) | 8.5 |
| I-E | 100% Capryl Pyrrolidone $(C_8P)$[1] | 7.7 |
| I-F | 100% Lauryl Pyrrolidone $(C_{12}P)$[2] | 22.9 |
| I-G | 100% Myristoleyl Pyrrolidone $(C_{14}P)$[3] | 52.6 |
| I-H | 100% Oleyl Pyrrolidone $(C_{18}P)$[4] | ---- |

1 = N-Octyl Pyrrolidone
2 = N-Dodceyl Pyrrolidone
3 = N-11-Tetradecenyl Pyrrolidone
4 = N-9-Octadecene Pyrrolidone

The above data show that neither water, ethanol or equal combinations of water and ethanol provide any significant enhancement effect upon pindolol. Methyl laurate and the pyrrolidones, particularly the $C_{12}$ and $C_{14}$ pyrrolidones, showed some significant enhancement of pindolol.

EXAMPLE II

A series of tests similar to Example I were conducted utilizing pindolol as the active agent with solutions containing 99% water and 1% of various the various pyrrolidone enchancer components. Test II-E also shows the results of using aqueous methyl laurate solutions. The results are as follows:

| Test No. | Enhancer Ingredient | | Flux [ug/cm2/hour] |
|---|---|---|---|
| II-A | 99%/1% | $H_2O/C_8P$ | 34.9 |
| II-B | 99%/1% | $H_2O/C_{12}P$ | 65.7 |
| II-C | 99%/1% | $H_2O/C_{14}P$ | 48.6 |
| II-D | 99%/1% | $H_2O/C_{18}P$ | 2.1 |
| II-E | 99%/1% | $H_2O/ML$ | 10.1 |

These tests show that 1% of the enhancer components in 99% water demonstrated comparable pindolol flux to that obtained by using 100% concentrations of the enhancer components. Comparable data with oleyl pyrrolidone is not available.

EXAMPLE III Again, following the procedure as in the preceding examples, the pindolol flux was obtained using 50% ethanol, 49% water and 1% pyrrolidone enhancer combinations. The data of Tests I-A, I-B and I-C is included for comparative purposes. The results are:

| Test No. | Enhancer Ingredient | | Flux [ug/cm2/hour] |
|---|---|---|---|
| I-A | 100% | Ethanol | 3.5 |
| I-B | 100% | Water | 1.6 |
| I-C | 50%/50% | EtOH/Water | 3.9 |
| III-A | 50%/49%/1% | $ETOH/H_2O/C_8P$ | 17.3 |
| III-B | 50%/49%/1% | $EtOH/H_2O/C_{12}P$ | 163.3 |
| III-C | 50%/49%/1% | $EtOH/H_2O/C_{14}P$ | 132.2 |
| III-D | 50%/49%/1% | $EtOH/H_2O/C_{18}P$ | 125.8 |

These tests show that, as compared with Example II, by replacing a 99% water/1% NAP solution with a 50% ethanol/49% water/1% NAP solution, the pindolol flux increases dramatically in most cases. These results are clearly unexpected as Tests I-A and I-C show that equal concentrations of ethanol and water, without the presence of NAP, provided virtually the same pindolol flux as did ethanol alone. Hence, one would not expect that the addition of NAP to water/ethanol solutions would increase pindolol flux so significantly over NAP/water solutions.

EXAMPLE IV

The procedure of Example III was followed with the exception that 1% methyl laurate (ML) was added to the enhancer formulations as a cell-envelope disordering compound replacing 1% water. The pindolol flux was as follows:

| Test No. | Enhancer Ingredient | | Flux [ug/cm2/hour] |
|---|---|---|---|
| IV-A | 50%/48%/1%/1% | ETOH/$H_2O$/ML/$C_8P$ | 146.1 |
| IV-B | 50%/49%/1%/1% | EtOH/$H_2O$/ML/$C_{12}P$ | 342.2 |
| IV-C | 50%/48%/1%/1%% | EtOH/$H_2O$/ML/$C_{14}P$ | 855.3 |
| IV-D | 50%/48%/1%/1% | EtOH/$H_2O$/ML/$C_{18}P$ | 219.2 |

These tests show that, as compared with Example III, by adding 1% of methyl laurate as a cell-envelope disordering compound to the pyrrolidone-ethanol-water enhancer, the pindolol flux again increases dramatically in all cases including capryl pyrrolidone. These results are clearly unexpected as Test II-E shows that use of methyl laurate, as compared to N-alkyl or alkenyl pyrrolidones, in water is generally inferior.

The following examples essentially duplicate Examples I through IV except a less water soluble permeant, estradiol, was used as the active ingredient instead of pindolol.

EXAMPLE V

This example shows the estradiol flux obtained using a saturated solution of estradiol in the individual enhancer components separately and, in the case of water and ethanol, in combination.

| Test No. | Enhancer Ingredient | Flux [ug/cm2/hour] |
|---|---|---|
| V-A | 100% Ethanol | 0.68 |
| V-B | 100% Water | — |
| V-C | 50%/50% Ethanol/Water | 0.57 |
| V-D | 100% Methyl Laurate (ML) | 0.33 |
| V-E | 100% Capryl Pyrrolidone ($C_8P$) | 0.15 |
| V-F | 100% Lauryl Pyrrolidone ($C_{12}P$) | 0.29 |
| V-G | 100% Myristoleyl Pyrrolidone ($C_{14}P$) | 0.19 |
| V-H | 100% Oleyl Pyrrolidone ($C_{18}P$) | ---- |

The above data show that neither ethanol or equal combinations of water and ethanol provide any significant enhancement effect upon estradiol. However, methyl laurate and the pyrrolidones showed even less enhancement of estradiol than ethanol or ethanol-water combinations.

EXAMPLE VI

A series of tests similar to Example V were conducted utilizing estradiol as the active agent with solutions containing 99% water and 1% of the various pyrrolidone enhancer components. Test VI-E also shows the results of using aqueous methyl laurate solutions. The results are as follows:

| Test No. | Enhancer Ingredient | | Flux [ug/cm2/hour] |
|---|---|---|---|
| VI-A | 99%/1% | $H_2O/C_8P$ | 0.83 |
| VI-B | 99%/1% | $H_2O/C_{12}P$ | 0.44 |
| VI-C | 99%/1% | $H_2O/C_{14}P$ | 0.40 |
| VI-D | 99%/1% | $H_2O/C_{18}P$ | 0.23 |
| VI-E | 99%/1% | $H_2O/ML$ | 0.27 |

Consistent with Example II, these tests show that 1% of the enhancer components in 99% water demonstrated comparable estradiol flux to that obtained when using 100% concentrations of the enhancer components as shown in Example V. The flux was generally better in the case of the pyrrolidones and lower in the case of methyl laurate.

EXAMPLE VII

Again, following the procedure as in Examples V and VI, the estradiol flux was obtained using 50% ethanol, 49% water and 1% pyrrolidone enhancer combinations. The data of Tests V-A and V-C is included for comparative purposes. The results are as follows:

| Test No. | Enhancer Ingredient | | Flux [ug/cm2/hour] |
|---|---|---|---|
| V-A | 100% | Ethanol | 0.68 |
| V-C | 50%/50% | EtOH/Water | 0.57 |
| VII-A | 50%/49%/1% | ETOH/$H_2O$/$C_8P$ | 1.47 |
| VII-B | 50%/49%/1% | EtOH/$H_2O$/$C_{12}P$ | 1.20 |
| VII-C | 50%/49%/1% | EtOH/$H_2O$/$C_{14}P$ | 1.28 |
| VII-D | 50%/49%/1% | EtOH/$H_2O$/$C_{18}P$ | 1.33 |

These tests show that, as compared with Example VI, by replacing a 99% water solution with a 50% ethanol/49% water/1% NAP solution, the estradiol flux increases dramatically in all cases. These results are clearly unexpected as Tests V-A and V-C show that equal concentrations of ethanol and water, without the presence of NAP, provided almost the same estradiol flux as did ethanol alone. Hence, one would not expect that the addition of NAP to water/ethanol solutions would increase estradiol flux so significantly over NAP/water solutions.

EXAMPLE VIII

The procedure of Example VII was followed with the exception that 1% methyl laurate (ML) was added to the enhancer formulations as a cell-envelope disordering compound replacing 1% water. The estradiol flux was as follows:

| Test No. | Enhancer Ingredient | | Flux [ug/cm2/hour] |
|---|---|---|---|
| VIII-A | 50%/48%/1%/1% | ETOH/$H_2O$/ML/$C_8P$ | 1.65 |
| VIII-B | 50%/48%/1%/1% | EtOH/$H_2O$/ML/$C_{12}P$ | 1.43 |
| VIII-C | 50%/48%/1%/1%% | EtOH/$H_2O$/ML/$C_{14}P$ | 1.23 |
| VIII-D | 50%/48%/1%/1% | EtOH/$H_2O$/ML/$C_{18}P$ | 1.18 |

These tests show that by adding 1% of methyl laurate as a cell-envelope disordering compound to the pyrrolidone-ethanol-water enhancer, the estradiol flux was comparable to the results shown in Example VII.

EXAMPLE IX

The following are exemplary of other compositions which can be formulated within the scope of this invention. However, they are illustrative only and are not intended to define the scope of the invention. The compositions can be conveniently formulated simply by mixing all components thoroughly. In some formulations, exact percentages are given whereas others are expressed by ranges. All compositions are in

percent by weight.

## FORMULATION IX-A

| Testosterone | 0.1- 1.0% |
| Enhancer | 99.0-99.9% |
| $C_8$ Pyrrolidone | 0.1- 5.0% |
| Ethanol | 5.0-70.0% |
| Water | 5.0-60.0% |

## FORMULATION IX-B

| Testosterone | 0.1- 1.0% |
| Enhancer | 99.0-99.9% |
| $C_{12}$ Pyrrolidone | 0.1- 5.0% |
| Ethanol | 5.0-70.0% |
| Water | 5.0-60.0% |
| Methyl laurate | 0.1- 5.0% |

## FORMULATION IX-C

| Progesterone | 0.1- 5.0% |
| Enhancer | 95.0-99.9% |
| $C_8$ Pyrrolidone | 0.1- 5.0% |
| Ethanol | 5.0-60.0% |
| Water | 5.0-70.0% |

## FORMULATION IX-D

| Progesterone | 0.1- 5.0% |
| Enhancer | 95.0-99.9% |
| $C_{14}$ Pyrrolidone | 0.1- 5.0% |
| Ethanol | 5.0-50.0% |
| Water | 5.0-70.0% |
| Glycerol monooleate | 0.1- 5.0% |

## FORMULATION IX-E

| | |
|---|---|
| Nicardipine | 0.1-20.0% |
| Enhancer | 80.0-99.9% |
| $C_{18}$ Pyrrolidone | 0.1- 5.0% |
| Ethanol | 5.0-60.0% |
| Water | 5.0-70.0% |

## FORMULATION IX-F

| | |
|---|---|
| Nicardipine HCl | 0.1-20.0% |
| Enhancer | 80.0-99.9% |
| $C_{14}$ Pyrrolidone | 0.1- 5.0% |
| Ethanol | 5.0-70.0% |
| Water | 5.0-50.0% |

## FORMULATION IX-G

| | |
|---|---|
| Propranolol HCl | 0.1-20.0% |
| Enhancer | 80.0-99.9% |
| $C_8$ Pyrrolidone | 0.1- 5.0% |
| Ethanol | 5.0-70.0% |
| Water | 5.0-70.0% |

## FORMULATION IX-H

| | |
|---|---|
| Nystatin | 0.1-10.0% |
| Enhancer | 90.0-99.9% |
| $C_{18}$ Pyrrolidone | 0.1- 5.0% |
| Ethanol | 5.0-70.0% |
| Water | 5.0-70.0% |

## FORMULATION IX-I

| | |
|---|---|
| Fentanyl | 0.1-10.0% |
| Enhancer | 90.0-99.9% |
| $C_{12}$ Pyrrolidone | 0.1- 5.0% |
| Etanol | 5.0-70.0% |
| Water | 5.0-70.0% |

## FORMULATION IX-J

| | |
|---|---|
| Fentanyl Citrate | 0.1-10.0% |
| Enhancer | 90.0-99.9% |
| $C_{12}$ Pyrrolidone | 0.1- 5.0% |
| Ethanol | 5.0-70.0% |
| Water | 5.0-70.0% |
| Methyl Laurate | 0.1- 5.0% |

EP 0 491 076 A1

### FORMULATION IX-K

| | |
|---|---|
| Oxymorphone | 0.01-10.0% |
| Enhancer | 90.0-99.99% |
| $C_{18}$ Pyrrolidone | 0.1- 5.0% |
| Ethanol | 5.0-70.0% |
| Water | 5.0-70.0% |

### FORMULATION IX-L

| | |
|---|---|
| Levorphanol | 0.01-10.0% |
| Enhancer | 90.0-99.99% |
| $C_8$ Pyrrolidone | 0.1- 5.0% |
| Ethanol | 5.0-60.0% |
| Water | 5.0-60.0% |
| Oleic Acid | 0.1- 5.0% |

### FORMULATION IX-M

| | |
|---|---|
| Haloperidol | 0.1-10.0% |
| Enhancer | 90.0-99.9% |
| $C_{22}$ Pyrrolidone | 0.1- 5.0% |
| Ethanol | 5.0-70.0% |
| Water | 5.0-70.0% |

### FORMULATION IX-N

| | |
|---|---|
| Metoclopramide | 0.1-10.0% |
| Enhancer | 90.0-99.9% |
| $C_{14}$ Pyrrolidone | 0.1- 5.0% |
| Ethanol | 5.0-70.0% |
| Water | 5.0-70.0% |
| Glycerol Dioleate | 0.1- 5.0% |

### FORMULATION IX-O

| | |
|---|---|
| Isosorbide Dinitrate | 0.1-15.0% |
| Enhancer | 85.0-99.9% |
| $C_{12}$ Pyrrolidone | 0.1- 5.0% |
| Ethanol | 5.0-70.0% |
| Water | 5.0-70.0% |

### FORMULATION IX-P

| | |
|---|---|
| Ergotamine Tartrate | 0.1-10.0% |
| Enhancer | 90.0-99.9% |
| $C_{18}$ Pyrrolidone | 0.1- 5.0% |
| Ethanol | 5.0-70.0% |
| Water | 5.0-70.0% |

14

FORMULATION IX-Q

| | |
|---|---|
| Bromocriptine Mesylate | 0.1-10.0% |
| Enhancer | 90.0-99.9% |
| $C_{12}$ Pyrrolidone | 0.1- 5.0% |
| Ethanol | 5.0-70.0% |
| Water | 5.0-70.0% |
| Methyl Laurate | 0.1- 5.0% |

FORMULATION IX-R

| | |
|---|---|
| Leuprolide | 0.0001- 2.0% |
| Enhancer | 98.0-99.9999% |
| $C_{12}$ Pyrrolidone | 1.0% |
| Ethanol | 50.0% |
| Water | 48.0% |
| Methyl Laurate | 1.0% |

FORMULATION IX-S

| | |
|---|---|
| Hydrocortisone | 0.01-10.0% |
| Enhancer | 90.0-99.99% |
| $C_{14}$ Pyrrolidone | 1.0% |
| Ethanol | 50.0% |
| Water | 48.0% |
| Methyl Laurate | 1.0% |

FORMULATION IX-T

| | |
|---|---|
| Calcitriol | 0.0001- 0.01% |
| Enhancer | 99.99-99.9999% |
| $C_{12}$ Pyrrolidone | 1.0% |
| Ethanol | 65.0% |
| Water | 33.0% |
| Methyl Laurate | 1.0% |

FORMULATION IX-U

| | |
|---|---|
| Albuterol Sulfate | 0.1-10.0% |
| Enhancer | 90.0-99.9% |
| $C_{14}$ Pyrrolidone | 2.0% |
| Ethanol | 45.0% |
| Water | 53.0% |

FORMULATION IX-V

| | |
|---|---|
| Desmopressin | 0.0001- 2.0% |
| Enhancer | 98.0-99.9999% |
| $C_{12}$ Pyrrolidone | 1.0% |
| Ethanol | 50.0% |
| Water | 48.0% |
| Methyl Laurate | 1.0% |

### FORMULATION IX-W

| | |
|---|---|
| Ketotifen Fumerate | 0.01-10.0% |
| Enhancer | 90.0-99.99% |
| $C_{14}$ Pyrrolidone | 2.0% |
| Ethanol | 30.0% |
| Water | 67.0% |
| Glycerol Monolaurate | 1.0% |

### FORMULATION IX-X

| | |
|---|---|
| Medroxyprogesterone Acetate | 0.001- 2.0% |
| Enhancer | 98.0-99.999% |
| $C_{14}$ Pyrrolidone | 1.0% |
| Ethanol | 60.0% |
| Water | 37.0% |
| Glycerol Monooleate | 2.0% |

### FORMULATION IX-Y

| | |
|---|---|
| Piroxicam | 0.01- 5.0% |
| Enhancer | 95.0-99.99% |
| $C_{12}$ Pyrrolidone | 2.0% |
| Ethanol | 45.0% |
| Water | 51.0% |
| Glycerol Monooleate | 2.0% |

### FORMULATION IX-Z

| | |
|---|---|
| Leuprolide Acetate | 0.001- 1.0% |
| Enhancer | 99.0-99.999% |
| $C_{14}$ Pyrrolidone | 1.0% |
| Ethanol | 50.0% |
| Water | 48.0% |
| Methyl Laurate | 1.0% |

While the above examples illustrate numerous embodiments of the invention, the scope is limited only by the operability exhibited by improved enhancement of permeants attributable to the enhancer combination of $C_8$ to $C_{22}$ N-alkyl or alkenyl pyrrolidones, $C_2$ and $C_3$ alkanols and inert carriers, with or without cell-envelope disordering compounds or added optional ingredients. It is to active permeants contained in the enhancer composition that this invention is drawn. Compositions comprising active ingredients and enhancers in unit dosage forms contained in sustained release devices such as patches, as previously referred to, are considered to be within the scope of the invention. Such sustained release devices are brought into communication with a predetermined area of the skin and may be applied for a predetermined period of time. Generally, the composition will be contained in a reservoir in the device and be delivered to the skin from the device. In this manner the amount of drug and the delivery period can be determined. In view of the above, the invention is, therefore, limited in scope only by the appended claims and their functional equivalents.

### Claims

1. A pharmaceutical composition for topical application having penetration-enhancing properties comprising:
   (a) a safe and effective amount of an active pharmaceutical permeant contained in,
   (b) a penetration-enhancing vehicle comprising.

16

(i) about 0.01 to about 20.0 % by weight of an N-aliphatic pyrrolidone wherein the aliphatic group is a $C_8$ to $C_{22}$ alkyl or alkenyl group which may be straight or branched chained and mixtures thereof;

(ii) about 5.0 to about 70.0 % by weight of a lower alkanol selected from the group consisting of ethanol, propanol and isopropanol and mixtures thereof; and

(iii) about 5.0 to about 70.00 % by weight of an inert carrier.

2. A composition according to Claim 1 wherein the N-aliphatic pyrrolidone is present in amounts ranging between about 0.1 and 5% by weight.

3. A composition according to Claim 2 wherein the N-aliphatic pyrrolidone is a member selected from the group consisting of N-capryl pyrrolidone, N-lauryl pyrrolidone, N-oleyl pyrrolidone and N-myristoleyl pyrrolidone.

4. A composition according to Claim 3 wherein the inert carrier is water.

5. A composition according to Claim 1-4 wherein the penetration enhancing vehicle additionally contains between about 0.01 and 20.0 % w. of a cell-envelope disordering compound.

6. A composition according to Claim 5 wherein the cell-envelope disordering compound and N-aliphatic pyrrolidone are each present in amounts ranging between about 0.1 to 2.0 %w., and wherein the cell-envelope disordering compound is a member selected from the group consisting of methyl laurate, glycerol monolaurate, oleic acid, oleyl alcohol, glycerol monooleate, glycerol dioleate, glycerol trioleate and mixtures thereof

7. A unit dosage form for transdermally administering an active pharmaceutical permeant to a predetermined area of skin comprising a sustained release device adapted to communicate with said area of skin having contained therein a pharmaceutical composition according to any of Claims 1-6.

8. A method for enhancing the penetration of an active pharmaceutical permeant through the skin of a human or warm-blooded animal which comprises applying to the skin a composition comprising:

(a) a safe and effective amount of an active pharmaceutical permeant contained in,

(b) a penetration-enhancing vehicle comprising.

(i) about 0.01 to about 20.0 % by weight of an N-aliphatic pyrrolidone wherein the aliphatic group is a $C_8$ to $C_{22}$ alkyl or alkenyl group which may be straight or branched chained and mixtures thereof;

(ii) about 5.0 to about 70.0 % by weight of a lower alkanol selected from the group consisting of ethanol, propanol and isopropanol and mixtures thereof; and

(iii) about 5.0 to about 70.00 % by weight of an inert carrier.

9. A method according to Claim 8 wherein the N-aliphatic pyrrolidone is present in amounts ranging between about 0.1 and 5% by weight.

10. A method according to Claim 9 wherein the N-aliphatic pyrrolidone is a member selected from the group consisting of N-capryl pyrrolidone, N-lauryl pyrrolidone, N-oleyl pyrrolidone and N-myristoleyl pyrrolidone.

11. A method according to Claim 10 wherein the inert carrier is water.

12. A method according to Claims 8-11 wherein the penetration enhancing vehicle additionally contains between about 0.01 and 20.0 % w. of a cell-envelope disordering compound.

13. A method according to Claim 12 wherein the cell-envelope disordering compound and N-aliphatic pyrrolidone are each present in amounts ranging betweeen about 0.1 and 2.0 %w. and wherein the cell-envelope disordering compounds is a member selected from the group consisting of methyl laurate, glycerol monolaurate, oleic acid, oleyl alcohol, glycerol monooleate, glycerol dioleate, glycerol trioleate and mixtures thereof.

**14.** A method for transdermally administering an active pharmaceutical permeant to a predetermined area of skin in unit dosage form comprising applying to the predetermined area of skin a sustained release device having contained therein a pharmaceutical composition according to any of Claims 8-13.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 704 346 (JAVITT et al.) <br> * Claims 1,5-7; page 14, lines 17-23; page 15, lines 21-24; pages 18,19, example 1; page 23, example 3, in particular lines 5-10,27 * <br> --- | 1-5,7-12,14 | A 61 K 47/10 <br> A 61 K 47/22 |
| Y | EP-A-0 306 192 (ELI LILLY AND CO.) <br> * Claim 1; pages 4,5, example 1 * <br> --- | 1-4,7-11,14 | |
| Y | EP-A-0 129 285 (THE PROCTER & GAMBLE CO.) <br> * Claim 1; page 50, example 16 * & US-A-4 537 776 (Kat. D) | 5,12 | |
| A | ----- | 6,13 | |

TECHNICAL FIELDS SEARCHED (Int. Cl.5)

A 61 K

The present search report has heen drawn up for all claims

| Place of search | Date of completion of the search | Examiner |
|---|---|---|
| THE HAGUE | 11-06-1991 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P0401)